(19) Europäisches Patentamt / European Patent Office / Office européen des brevets

(11) **EP 4 786 956 A1**

(12) **EUROPEAN PATENT APPLICATION**

(43) Date of publication:
05.08.2026 Bulletin 2026/32

(51) International Patent Classification (IPC):
G01N 21/45 $^{(2006.01)}$   G01N 21/552 $^{(2014.01)}$
G01N 21/77 $^{(2006.01)}$   G01N 33/543 $^{(2006.01)}$
G02B 6/122 $^{(2006.01)}$   G01B 9/00 $^{(2006.01)}$

(21) Application number: 25155292.3

(22) Date of filing: 31.01.2025

(52) Cooperative Patent Classification (CPC):
G01N 21/7703; G01N 21/45; G01N 21/553;
G01N 33/54373; G02B 6/1226; G01N 2021/458;
G01N 2021/7776; G01N 2021/7779

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 30.01.2025 PL 45109025

(71) Applicant: POLITECHNIKA WARSZAWSKA
00-661 Warszawa (PL)

(72) Inventor: Gosciniak, Jacek
01-887 Warszawa (PL)

(74) Representative: Rybarczyk, Dariusz Pawel et al
Polservice
Kancelaria Rzecznikow
Patentowych sp. z o.o.
Bobrowiecka 8
00-728 Warszawa (PL)

(54) **INTEGRATED PLASMO-PHOTONIC SENSOR DEVICE WITH VOLTAGE CONTROLED DETECTION AND APPARATUS COMPRISING AN ARRAY OF SUCH SENSOR DEVICES**

(57) The disclosure relates to a device comprising a first photonic waveguide (101) providing light to a plasmonic slot waveguide constituted of two metal electrodes (104, 105) serving as a plasmonic Mach-Zehnder Interferometer (MZI) sensor, a second photonic waveguide (109) that collects light from a MZI-based plasmonic sensor. It further comprises an overall chip (110), optical coupling structures (102, 107) at both ends of the sensor acting as the optical I/Os; and an optical splitter (103) and an optical combiner (108) for optical splitting at a first junction (103) of the plasmonic slot MZI and optical combining at a second junction (108) of the plasmonic slot MZI. It further comprises a DC voltage or current source (106) connected between the first electrode (104) and the second electrode (105) that constitute the plasmonic slot MZI. The disclosure relates also to an apparatus comprising an array of such devices.

Fig. 1

**Description**

**FIELD OF THE INVENTION**

The present invention relates to a device comprising planar integrated photonic sensors and biosensors and more specifically to a monolithic co-integration of CMOS photonics and plasmonic components into an optimally biased plasmonic slot Mach-Zehnder Interferometer as a means to increase the sensors sensitivity to unprecedented levels and at low manufacturing cost. The present invention relates furthermore to an apparatus comprising an array of such sensor devices.

**PRIOR ART**

In recent years, integrated photonic sensors have attracted a lot of attention due to their high-sensitivity, label-free and real-time operation and the potential of mass production at low cost. The mechanism of operation of the waveguide-integrated photonic sensors typically relies on the interaction of the evanescent field of the guided optical mode with the analyte molecules placed on top of the transducing photonic waveguide, and detection is ultimately bound by the propagation loss of the waveguide. However, the performance of such sensors is limited by a weak evanescent field from the photonic waveguide. In consequence, a long sensing arm is needed that often exceeds several millimeters or even centimeters what affects its compactness and restricts its applications. One of the solution relies on replacing the photonic waveguide by its plasmonic counterpart. As a result, a significant electric field enhancement can be achieved what translates on a stronger interaction of the electromagnetic field with the analyte and, in consequence, shorter interaction length as it enables the interaction of the entire plasmonic mode with the surrounding analyte. Simultaneously, to mitigate high propagation losses of the plasmonic structure, the plasmonic waveguides can be co-integrated with low-loss photonic waveguides taking in this way the advantages of both technologies.

Sensing mechanism relies on the comparison of a difference in propagation of light through the photonic or plasmonic waveguide under the absence and presence of the analyte under test. However, such a simple device requires sequential reference and sensing measurements. To reduce the time-dependent measurement errors such as drift or fluctuations in light source power, an effective referencing strategy can be taken into account where the ratio of the transmission through two arms is considered. Alternatively, the light can be split between the reference and sensing arms while a pair of detectors can be used to continuously measure the signals from both arms. To reduce complexity, the sensing mechanism can operate based on detecting any refractive index change that occurs between both arms. In consequence, a Mach-Zehnder Interferometer (MZI) can be considered where the refractive index change induces a phase change on the propagating photonic or plasmonic modes between both MZI arms. The MZI translates this phase change into a spectral shift of the MZI resonance. Compared to the photonic sensors, the plasmonic counterparts offer higher sensitivity values as the analyte can be placed directly on a metal electrode, i.e., in the maximum electric field of the operating mode, thus, enhancing the interaction of the electromagnetic radiation with the analyte. Simultaneously, the inevitable high propagation plasmonic losses can be counterbalanced by a co-integration of plasmonic structures that serve as a transducer with low-loss photonic waveguides that deliver light to the plasmonic transducer and then collect it from a sensor. Through such an integration the miniaturized photonic integrated circuit (PIC) for sensing applications can be delivered.

The main goal of each sensor is to maximize the sensitivity to refractive index changes and to maximize the extinction ratio (ER) at the output of the sensor while minimizing the overall losses. As such, the proposed sensor arrangement can provide much higher sensitivity due to placing sensing materials, ions in the maximum of the propagating mode by applying a voltage between metal electrodes that forces ions to drift towards one of the electrode edges i.e., at the maximum of the propagating mode or by placing an ion-exchange layer/membrane at a side of one or both electrodes. Furthermore, the above proposal allows to maximize the coupling efficiency from the photonic waveguide to the plasmonic part as well as it a screening the photonic part of the sensor by placing it in the substrate below what enables the evanescent coupling between the photonic waveguide and the plasmonic section. In this way, the entire structure can be covered with a liquid analyte without the need of screening the photonic part from the liquid.

US 2020/003696 A1 discloses a device comprising a first optical Mach-Zehnder interferometric sensor with a large FSR, wherein a plasmonic waveguide thin-film or hybrid slot, is incorporated as transducer element planar integrated on Si3N4 photonic waveguides and a second optical interferometric Mach-Zehnder, both comprising thermo-optic phase shifters for optimally biasing said MZI sensor and MZI as variable optical attenuator VOA.

US 2011/0188047 A1 discloses a sensor and corresponding method for sensing variations in a parameter which employ an optical device defining two optical paths differentially affected by a variation in the parameter so as to change the differential phase between the two paths.

US 2018/340884 A1 discloses an infrared sensor and a method of detecting molecular species in a liquid.

Reference is also made to the following publications:

1. M. P. Nielsen, L. Lafone, A. Rakovich, T. P. H. Sidiropoulos. M. Rahmani, S. A. Maier, and R. F. Oulton, "Adiabatic Nanofocusing in Hybrid Gap Plasmon Waveguides on the Silicon-on-Insulator Platform," Nano Lett. 2016, 16(2), 1410-1414.

2. O. Parriaux, and G. J. Veldhuis, "Normalized Analysis for the Sensitivity Optimization on Integrated Optical Evanescent-Wave Sensors," J. of Lightwave Tech. 1998, 16(4), 573-582.

3. K. Fotiadis et al., "High-Sensitivity Bimodal Plasmo-Photonic Refractive Index Sensor," ACS Photonics 2023, 10, 2580-2588.

4. B. Hinkov, M. David, G. Strasser, B. Schwarz, and B. Lendl, "On-chip liquid sensing using mid-IR plasmonics," Front. Photonics 2023, 4:1213434.

5. C. J. Mitchell, T. Hu, S. Sun, C. J. Stirling, M. Nedeljkovic, A. C. Peacock, G. T. Reed, G. Mashanovich, and D. J. Rowe, "Mid-infrared silicon photonics: From benchtop to real-world applications," APL Photon. 2024, 9, 080901.

6. K. Fotiadis et al., "High-Sensitivity Bimodal Plasmo-Photonic Refractive Index Sensor," ACS Photonics 2023, 10, 2580-2588.

Aim of the Invention

The present invention aims to address the various issues indicated above simultaneously by proposing an ultra-sensitive sensor and biosensor device that can be integrated in a micrometer scale chip-based configuration by using simple and low-cost fabrication methods, such as those based on standard CMOS-compatible material platform.

## Summary

The invention relates to a photonic sensor device as defined in independent claim 1. Dependent claims 2-12 relate to individual embodiments of the invention defined in claim 1. The invention relates also to an apparatus comprising an array of such sensor devices defined in claim 13. Dependent claims 14-17 relate to individual embodiments of the apparatus defined in claim 13.

This invention contains three novel and inventive concepts of on-chip sensors and biosensors that allow to achieve high sensitivity at extremally short sensing length. The first novel/inventive concept relies on the utilizing the well-known plasmonic slot waveguide (metal-insulator-metal (MIM) waveguide) as a Mach-Zehnder Interferometer (MZI) where the opposite sides of metal electrodes forming a slot are used as MZI arms. In such an arrangement, light is provided to the MZI "splitter" by a photonic mode where the splitter consists of a tapered photonic waveguide coupled to the plasmonic slot waveguide. The second "splitter" that collects light from the MZI "arms" consists of a similar tapered photonic waveguide coupled to the plasmonic slot waveguide.

As with the other MZI-based sensors, the sensing mechanism involves a detection of refractive index changes in one of the MZI arms that is in contact with an analyte sample. This refractive index change induces a phase change of the sensitive plasmonic mode that propagates at this arm. The MZI translates this phase change into a wavelength shift at the interferometer output, i.e., in the photonic waveguide. As both MZI "arms" in the proposed sensor arrangement are in direct contact with the analyte sample, some separation mechanism is needed to separate ions present in the sample to introduce a high gradient distribution of ions in the sample. In consequence, a second inventive concept is proposed in this application.

The second inventive concept relies on an active separation technique that allows effective separation of chemical, biochemical, biological substances and/or molecules or other physical quantities present in liquids or gases or any biological samples that in the rest of the application are sometimes referred to as ions, in response to an electric field applied between metal electrodes that, simultaneously, constitute a plasmonic slot waveguide and thus it allows for stronger interaction of the plasmonic mode with ions present in the analyte. In the absence of electric field in the analyte (here liquid), the positively and negatively charged ions or any biological molecules are evenly distributed in the liquid solution. In consequence, the concentration of ions (or biological molecules) close to one of the metal surfaces, i.e., in the maximum of the propagating plasmonic mode, is very low, thus, the interaction of the plasmonic mode with the ions is reasonably weak and, consequently, the response of the system to the presence of the ions (or biological molecules) in the liquid is weak. However, under the applied DC voltage, the negative charged ions (anions or any biological molecules) move towards a positive electrode (anode), while the positive charged ions (cations or any biological molecules) move towards a negative electrode (cathode). Therefore, the ions are shifted to the maximum electric field of the propagating plasmonic mode, thus, the interaction of the mode with the ions is highly enhanced what translates on the stronger response of the sensor.

The third inventive concept relies on an alternative ion separation technique, the passive separation technique, implemented to this design that allows effective separation of negative or positive charges such as chemical, biochemical, biological substances and/or molecules or other physical quantities present in liquids or gases or any biological samples that in the rest of this specification are sometimes referred to as ions, in response to the presence of an ion-exchange layer (also referred to as the ion-separation layer or preconcentration layer in this specification) that is deposited on one or both of the electrodes inside a MIM gap. For a liquid containing some ions, anions or cations, the ion-exchange layer/membrane can serve as a surface that attracts ions of the opposite sign. For example, for a liquid containing $NO_2$ anions (negative charges) and some cations (positive charges), the net negative charge of the $NO_2$ anion attracts it to the anion-exchange layer/membrane causing the $NO_2$ anion to be absorbed by the layer/membrane, and for a thin layer of ion-exchange layer/membrane, to pass through it. Simultaneously, the net positive charge of the cation repels it from the anion-exchange layer/membrane, dissuading the cations from absorbing or passing through it. An opposite effect can be achieved with a cation-exchange layer/membrane that attracts and absorbs cations and repels anions. As in the case of an active separation method (with a DC voltage), the ions are transported to one or both electrodes, i.e., to the maximum electric field of the propagating plasmonic mode, thus, the interaction of the mode with the ions is highly enhanced what translates on the stronger response of the sensor as the optical characteristics of the light propagating through the MIM structure changes. Taking into account a small separation between metal electrodes that constitute a liquid channel, most of the ions present in the liquid under test will be attracted to the ion-exchange layer/membrane and thus participate in sensing.

The ion-exchange layer/membrane can be selected from the group comprising:

- an ion exchange resin
- metal-oxides
- metal-organic frameworks (MOFs).

Whenever the term "device" or "sensor device" or "sensor" is used in this specification and claims, it shall be understood as an overall set of interoperable components that make up the invention. The terms "photonic part" or "photonic section" and "plasmonic part" or "plasmonic section" represent each a component or a set of components selected and adapted for manipulating photons and plasmons, respectively. Within the photonic section/part, photonic/optical waveguides and fibers are used, among others, while within the plasmonic section/part, plasmonic waveguides or plasmonic slot waveguides are used, among others. The inventive combination of structures of both mentioned types is also referred to as a "hybrid plasmo-photonic slot waveguide" or using similar terminology throughout this specification. Moreover, the term "apparatus" used in this specification and claims shall be understood as an array of multiple sensor devices of the present invention. Furthermore, in the context of the present invention, the term "analyte" refers to a substance being tested by the sensor device of the present invention and can include a variety of substances including chemical, biochemical, biological substances and/or molecules or other physical quantities present in liquids or gases or any biological samples.

## BRIEF DESCRIPTION OF DRAWINGS

Embodiments of the present invention will now be described with reference to the accompanying drawings, wherein:

**Fig. 1** shows a cross-section (a), a perspective view (b) and a top view (c) of a first embodiment of a hybrid plasmo-photonic slot waveguide arranged in a Mach-Zehnder Interferometer schema used for a plasmonic section of a sensor, where a biased voltage is applied between straight electrodes;

**Fig. 2** shows a cross-section (a) and a perspective view (b) of a second embodiment of a hybrid plasmo-photonic slot waveguide arranged in a Mach-Zehnder Interferometer schema used for a plasmonic section of a sensor, where a buried photonic waveguide is used for light coupling;

**Fig. 3** shows a perspective view of a third embodiment of a hybrid plasmo-photonic slot waveguide arranged in a Mach-Zehnder Interferometer schema used for a plasmonic section of a sensor, where a grating coupler is used for light coupling from/to an optical fiber;

**Fig. 4** shows a cross-section of a simulated optical and direct current (DC) electrical fields of a plasmonic slot waveguide with an analyte core and Au electrodes; **Fig. 5** shows a perspective view (a) and top views (b, c) of a fourth embodiment of a hybrid plasmo-photonic slot waveguide arranged in a Mach-Zehnder Interferometer schema used for a plasmonic section of a sensor, where a biased voltage is applied between electrodes, one or both of which are curved;

**Fig. 6** shows a top view of a plasmonic slot waveguide with optical and direct current (DC) electric field distributions in an analyte under zero applied voltage (a) and certain applied voltage (b) with the characteristic ions distribution;

**Fig. 7** shows a top view of a plasmonic slot waveguide with optical electric field distributions in an analyte without (a) and with (b) preconcentration material deposited on one of the electrodes;

**Fig. 8** shows an example of a complex refractive indices of water and some chemical compounds such as (a) $(NH_4)_2SO_4$ and (b) $HNO_3$ with some characteristic absorption peaks corresponding to the maximum of the imaginary part of complex refractive indices;

**Fig. 9** shows cross-sections of a simulated in-plane optical electric field component of the propagating TE mode of a plasmonic slot waveguide with a thin layer of ions/molecules accumulated in one metal-water interface with water placed both in the gap and above a plasmonic structure;

**Fig. 10** shows a normalized transmission and mode effective index of a 150 $\mu$m long sensing arm of the Mach-Zehnder Interferometer as a function of the accumulation layer thickness, i.e., the ions thickness, for the Au electrode separation of 2 $\mu$m;

**Fig. 11** shows a top view of a photonic Mach-Zehnder Interferometer with a plasmonic slot waveguide arranged in the sensing arm and a phase shifter and a variable optical attenuator (VOA) arranged in the reference arm.

**Fig. 12** shows an on-chip integration of arrays of transducers and photodetectors in one apparatus for a sensing application.

## DETAILED DESCRIPTION

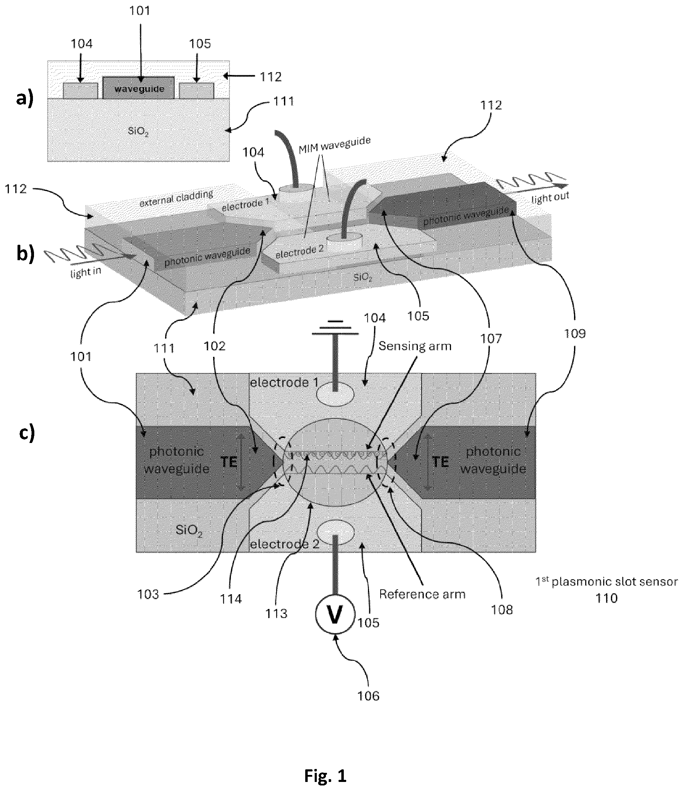

**Fig. 1** shows a cross-section (a), a perspective view (b) and a top view (c) of a first embodiment of a hybrid plasmo-photonic slot waveguide arranged in a Mach-Zehnder Interferometer schema. The hybrid plasmo-photonic slot waveguide includes a first photonic waveguide **101** terminated with a taper **102** providing light to a plasmonic slot waveguide constituted of two metal electrodes **104, 105** serving as a plasmonic Mach-Zehnder Interferometer (MZI) sensor; a second photonic waveguide **109** with a taper **107** that collects light from the MZI-based plasmonic sensor. The photonic waveguides can be made of any suitable material, such as, e.g., Si, SiN, Ge, $TiO_2$, $Al_2O_3$, and other materials known in the art. It further comprises an overall chip **110**, optical coupling structures **103, 108** at both ends of the sensor acting as optical input/output (I/Os); and an optical splitter **103** and an optical combiner **108** for optical splitting at the first junction **103** of the sensor (plasmonic slot MZI) and optical combining at the second junction **108** of the plasmonic slot MZI, respectively. The hybrid plasmo-photonic slot waveguide further comprises a DC voltage or current source **106** connected between a first metal contact (electrode) **104** and a second metal contact (electrode) **105** that constitute the plasmonic slot MZI. The opposite sides of the metal electrodes **104, 105** forming the slot are used as a sensing arm and a reference arm, respectively, as indicated in the figure. The mentioned structures were fabricated on a standard substrate made of a low refractive index material **111** such as $SiO_2$ in this example, and the photonic part of the chip (including the photonic waveguides **101, 109**) was screened from the environment by other low refractive index material **112** such as, e.g., $SiO_2$, polymers, etc., forming an external cladding over the photonic part. A sensing medium **113** was placed at the center of the slot with ions **114** separated under a voltage applied between the electrodes **104, 105** by the DC voltage source **106**.

In the context of this and the following embodiments of the invention, it is noted that the materials used for particular components of the invention are selected depending on the wavelength of light used, e.g. Si or SiN and $SiO_2$ are used for a wavelength of light of 1550 nm, while Ge and Si are used for mid-IR rage, just to mention few examples. One skilled in the art will be aware of other possible combinations of suitable materials. The selection of materials takes into consideration the required refractive index contrast (waveguide/surrounding material).

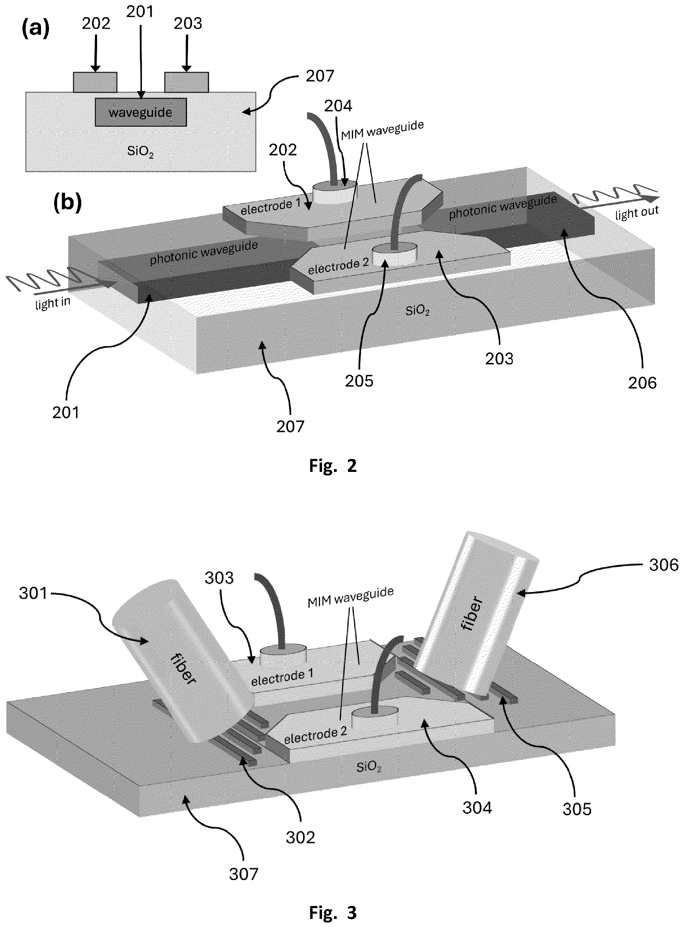

Referring now to **Fig. 2,** a cross-section (a) and a perspective view (b) of a second embodiment of a hybrid plasmo-photonic slot waveguide is shown. In many aspects, the second embodiment of the invention is similar to the first embodiment from **Fig. 1.** The hybrid plasmo-photonic slot waveguide in **Fig. 2** comprises a substrate **207**, two photonic waveguides **201, 206**, two electrodes **202, 203**, between which a voltage is applied by a DC voltage source. The photonic waveguides can be made of any suitable material, such as, e.g., Si, SiN, Ge, $TiO_2$, $Al_2O_3$ and other materials known in the art. The opposite sides of the electrodes **202, 203** define a plasmonic slot MZI. The second embodiment differs from the first embodiment in that the photonic waveguides **201, 206** in **Fig. 2** have no tapers and are buried in the substrate **207** to screen the photonic part from the environment.

In the second embodiment in **Fig. 2,** an evanescent coupling schema is used. The input photonic waveguide **201** and the output photonic waveguide **206** are each buried in the substrate **207** with a low refractive index (SiOz in this example), and the evanescent coupling takes place to and from the plasmonic slot waveguide constituted by the two metal electrodes **202, 203.** The metal electrodes **202, 203** serve both as the plasmonic materials supporting the MIM plasmonic slot mode and electrical electrodes with electrical contacts **204, 205** on top to apply the external power source.

Referring now to **Fig. 3,** a perspective view of a third embodiment of a hybrid plasmo-photonic slot waveguide is shown. On a low refractive index substrate **307** (SiOz in this example), two metal electrodes **303, 304** are arranged between which a voltage is applied by a DC voltage source. The opposite sides of the electrodes **303, 304** define a plasmonic slot MZI. Compared to the previously described embodiments, in the third embodiment in **Fig. 3** grating couplers **302, 305** are provided on both sides of the plasmonic slot MZI, each coupled with a respective fiber **301, 306.** In the case of the grating coupler arrangement presented in **Fig. 3,** light from the input fiber **301** couples through the grating coupler **302** to the plasmonic slot waveguide defined by opposite sides of the electrodes **303, 304** and then back through another grating coupler **305** to the collecting fiber **306.** According to the first to third embodiments of the invention above presented, three alternative coupling schemes are proposed. Light can be coupled into a plasmonic device either through the edge coupling scheme (**Fig. 1**) from the waveguide **101** provided on the substrate **111,** through the evanescent coupling (**Fig. 2**) from the waveguide **201** buried in the substrate **207,** or directly from the optical fiber **301** through the grating coupler **302 (Fig. 3),** what can make plasmonic sensors even more compact and integrable on a variety of substrates.

Considering the photonic waveguide coupling scheme, light can couple to the plasmonic section (slot plasmonic waveguide) either through a straight (no taper in the photonic waveguide in **Fig. 1**) or tapered photonic waveguide (**Fig. 1**) or evanescent coupling from a photonic waveguide buried in material below (**Fig. 2**). In the case of the tapered plasmonic waveguide, the excess losses as low as 0.5 dB can be achieved while for the evanescent coupling the efficiency up to 90 % can be expected for a perfectly fabricated device.

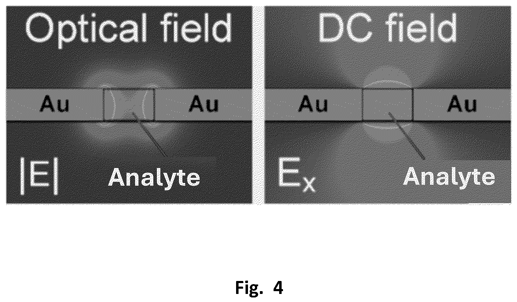

The proposed sensor that is based on the plasmonic slot waveguide offers a lot of benefits as the metal electrodes that support the plasmonic modes can serve simultaneously as high-speed electrodes what enables an optimal overlap between the electrical and optical fields (**Fig. 4**). The high electric field enhanced in the slot enables significant reduction of the sensor as a result of ions transfer to the side of one of the metal electrodes what, in turns, reduces both the contact resistance and the capacity in such device as well as enhances the interaction of light with ions under measurements. In consequence, the RC time constant drops what enables the increase of electrical bandwidth.

**Fig. 4** shows a cross-section of a simulated optical and direct current (DC) electrical fields of a plasmonic slot waveguide with an analyte core and Au electrodes. The metal electrodes can serve simultaneously as a plasmonic material supporting the propagating plasmonic modes and as high-speed electrical electrodes. As shown in **Fig. 4,** the optimal overlap between the optical and electrical fields is achieved. The electric field enhanced in the slot enables a significant reduction of the sensor by facilitating the transfer of ions to the side of one of the metal electrodes. This, in turn, reduces both the contact resistance and the capacity of the device, as well as enhances the interaction of light with ions during measurements. Consequently, the RC time constant decreases, thereby allowing for an increase in the electrical bandwidth.

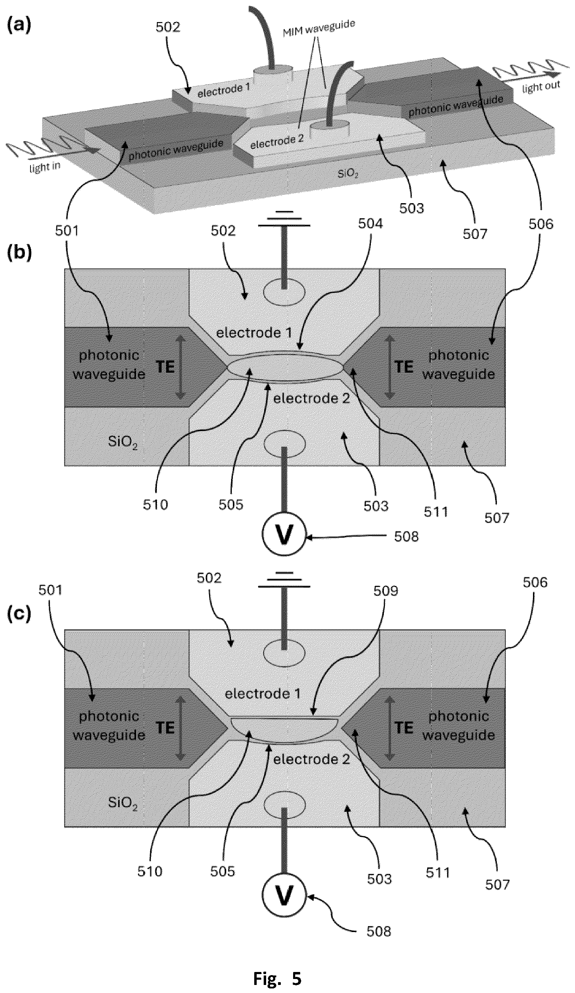

**Fig. 5** shows a perspective view (a) and top views (b, c) of a fourth embodiment of a hybrid plasmo-photonic slot waveguide. In many aspects, the fourth embodiment of the invention is similar to the first embodiment from **Fig. 1.** The hybrid plasmo-photonic slot waveguide in **Fig. 5** comprises a substrate **507,** two tapered photonic waveguides **501, 506,** two electrodes **502, 503,** between which a voltage is applied by a DC voltage source. The photonic waveguides can be made of any suitable material, such as, e.g., Si, SiN, Ge, $TiO_2$, $Al_2O_3$, etc. The opposite sides of the electrodes **502, 503** define a plasmonic slot MZI. In **Fig. 5a** and **b** both edges of the electrodes have curved edges, while in **Fig. 5c** only one edge is curved. In this way, a path length difference between both arms of the MZI can be introduced depending on specific requirements. The curvature of the edges can have a different values. The purpose of introducing electrode edge curvatures is to provide spatial separation of the two plasmonic modes propagating at the opposite edges of the electrodes to prevent them from interacting with each other.

The active plasmonic slot waveguide (MIM waveguide) presented in Fig. 5 in two variants can consist of two symmetric metallic contacts **502, 503** (**Fig. 5a** and **b**) or two asymmetric metallic contacts **502, 503** (**Fig. 5c**) with a sensing medium (analyte) **510** serving as the absorbing core. The coupled photons from the photonic waveguide **501** are converted into Surface Plasmon Polaritons (SPPs) that propagate along the MIM slot edges (**504**, **505** in **Fig. 5a,b** and **505, 509** in **Fig. 5c**) as two separated SPPs. For a small gap width, the two SPPs that propagate on the opposite edges of the metal contacts couple together giving rise to the well-known plasmonic gap mode that is characterized by an extreme mode confinement but, simultaneously, high losses that arise with the reduction of the gap width.

However, when the distance between opposite metal contact edges (**504**, **505** in **Fig. 5a, b** and **505, 509** in **Fig. 5c**) is efficiently high, the mode effective index approaches asymptotically the effective index of the SPP of a single metal-dielectric interface i.e., the slot plasmonic mode separates into two SPP modes that propagate on the opposite edges of the metal contacts (**504, 505** in **Fig. 5a, b and 505, 509** in **Fig. 5c**). As the photonic waveguides **501, 506** are designed to support only a fundamental TE mode, the coupled SPPs form two separated TM propagating modes (with the electric field perpendicular to the edges of the metal contact) in the plasmonic slot waveguide (**504, 505** in **Fig. 5a, b and 505, 509** in **Fig. 5c**). Finally, in a termination section of the metal structure **502, 503** both SPP modes couple back through a taper **511** to the photonic waveguide **506** creating a fundamental TE-polarized photonic mode. As observed above, the proposed structure behaves as a Mach-Zehnder interferometer with the MZI arms created by the metallic contacts **502, 503.** For an active separation technique with the DC voltage **508** (but the operation principles are the same for a passive separation technique with the ion-exchange layer/membrane), in the absence of the analyte **510** or in the presence of the analyte **510** but under a zero applied DC voltage **508,** the distribution of the ions/molecules that are embedded in the analyte that is placed in the gap, is uniform, thus, both SPPs experience the same attenuation and phase shift (under assumption of equal lengths of the MZI arms, i.e., for the same curvature of the metal edges). Thus, in the termination side of the plasmonic structure **502, 503** they couple back to the photonic waveguide **506** with the same phase and intensity. In consequence, a maximum power from the photonic waveguide **506** can be detected. By applying a bias DC voltage **508** between the two metal contacts **502, 503** and in the presence of the analyte **510** in the gap a uniform electric field is generated in the gap that separates the ions/molecules in a sensing medium in a direction of metal edges (**Fig. 4**).

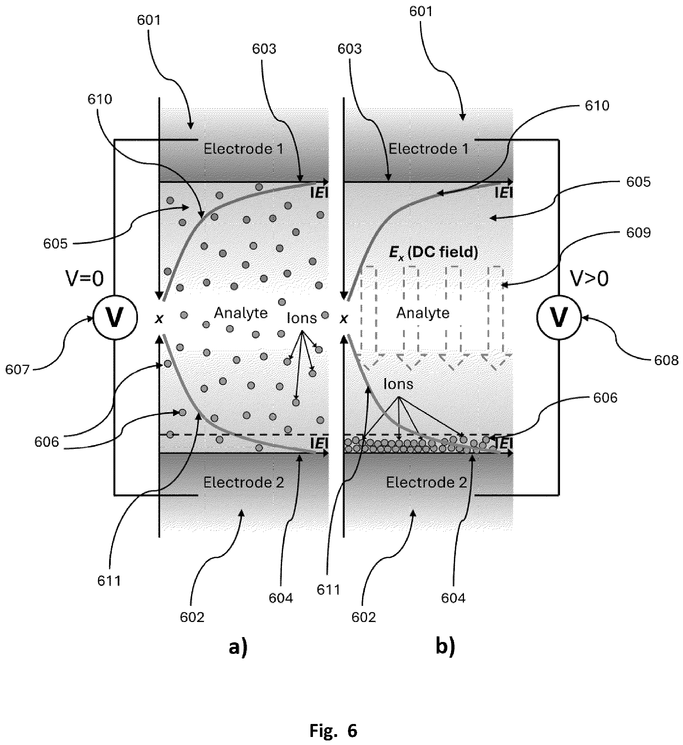

To provide a better understanding of the invention, the reference is now made to **Fig. 6** showing a top view of a plasmonic slot waveguide with optical **610, 611** and direct current (DC) electric field **609** distributions in an analyte **605** under zero applied voltage (a) and certain applied voltage (b) with the characteristic ions distribution. The optical electric fields **610, 611** reach maximum at the interface between metal electrode and the analyte and decays exponentially in a direction of the center of the gap. **Fig. 6** illustrates two electrodes **601, 602** of the plasmonic slot waveguide, which are connected to a DC voltage source **607, 608,** and between which a sensing medium (analyte) with ions/molecules **605** is provided. In the presence of the analyte **605** in the gap between the metal electrodes **601, 602** as it has been shown in **Fig. 6,** the electric field **609** generated in the gap separates positive and negative ions (anions and cations) **606** present in the analyte **605** in a direction of the opposite metal electrodes **601, 602.** Thus, the positive ions drift towards an edge **603** of the metal electrode **601** with a negative DC voltage **608** applied to it, while the negative ions drift towards an edge **604** of the metal electrode **602** with a positive DC voltage **608.** As a result, ions settle on one metal electrode edge **604** or in a very close proximity to this electrode edge **604**, while the second electrode edge **603** stays free of ions. In consequence, the MZI formed by two SPPs modes **610, 611** propagating on the opposite sides of the metal electrode edges **603, 604** is put in a state of unbalance as the ions **606** that deposited on the edge **604** of one electrode give a rise to the higher absorption and, simultaneously, induce the mode effective index change of the propagating mode associated with this specific arm, what induces a phase accumulation in this arm with respect to the second MZI arm. Thus, when both SPP modes **610, 611** couple back to the photonic waveguide (e.g. **506** in **Fig. 5**), the transmission from the MZI decreases. In the border case, when a $\pi$ phase shift is achieved between both SPP modes **610, 611** the transmission from the output photonic waveguide (e.g. **506** in **Fig. 5**) reaches minimum. In conclusion, the change of the mode effective index of one of the plasmonic modes **610, 611** that constitute the MZI arms associated with the presence of ions in the vicinity of this electrode edge results in a shift of the spectra resonances of the MZI-based sensor as it is highly sensitive to the sensing medium **605, 606** residing in the gap.

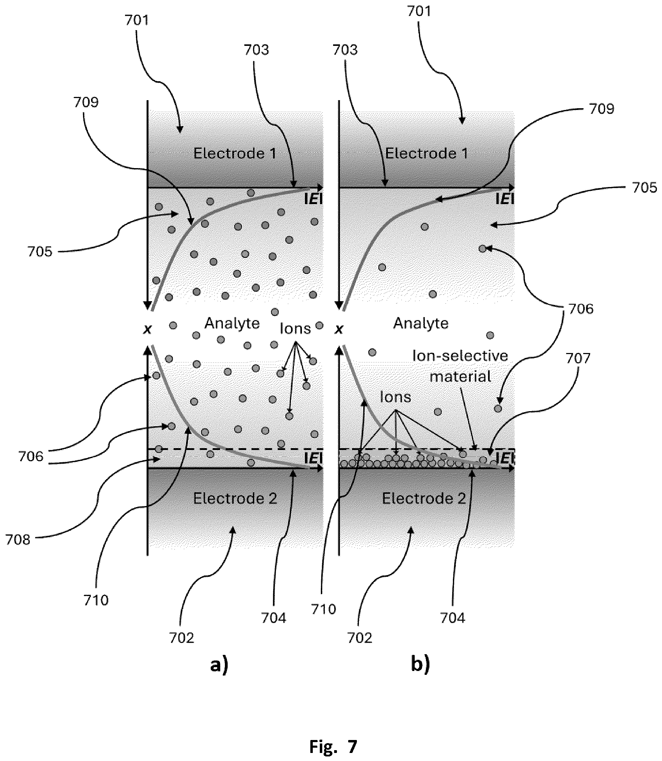

**Fig. 7** shows a similar situation as presented in **Fig. 6,** where, however, the separation between ions **706** is achieved through a deposition of the so-called ion-selective material **707** on one of the metal electrode edges **703, 704** that constitute the plasmonic slot waveguide formed by two metal electrodes **701, 702.** Compared to the previously presented method in **Fig. 6** that can be classified as an active method, the method in **Fig. 7** can be classified as a passive method as an ion-selective material is deposited on one of the metal electrode edges during the fabrication process. Furthermore, once deposited it cannot be avoided during further measurements. Thus, the MZI can be balanced only in the absence of the sensing medium with ions/molecules i.e., when the gap is empty. Once the sensing medium with ions/molecules is placed in the gap, the MZI is put in the state of unbalance that can be monitored by the photodetector placed at the termination of the MZI.

**Operation principles of the MZI-based sensors**

For non-resonant structures such as, for example, straight or spiral waveguides, the sensing mechanism relies on the interaction of the analyte molecules with the evanescent field of the guided optical mode and the sensitivity of such sensors is related to a propagation loss of the waveguide. For a $L$-long sensing waveguide that is characterized by initial losses of $\alpha$ related to the waveguide geometry (in the absence of the analyte), the change of transmitted light intensity $\Delta I$ due to the presence of an analyte is given by

$$\Delta I = exp(-\alpha L)[1 - exp(-\Gamma \alpha' L) \approx exp(-\alpha L)] \cdot \Gamma \alpha' L \qquad (1)$$

where $\alpha'$ denotes the absorption coefficient of the analyte and $\Gamma$ is the modal confinement factor in the analyte. In consequence, the sensing waveguide should be characterized by very low propagation losses within considered spectral band.

On the contrary, the spectral response of the proposed MZI, as well as other MZI-based resonant devices, varies with wavelength and depends on the beam intensity ratio between both propagating beams along the MZI arms and the phase difference between them, thus the output intensity is given by:

$$I_{out} = (A_1 + A_2)^2 = |A_1|^2 + |A_2|^2 + 2|A_1||A_2|cos(\Delta\phi) \tag{2}$$

where $I_i = |A_i|^2 exp(i2\varphi_i)$ and $\varphi_i = 2\pi n_i L/\lambda$ (for $i$=1, 2, i.e., for both arms of the MZI). Here, $A_i$ is the mode amplitude in one of the MZI arms, $n_i$ is the effective refractive index of the corresponding amplitude, $L$ is the length of the interferometer arm and $\lambda$ is the source wavelength. In consequence we receive:

$$I_{out} = (|A_1|^2 + |A_2|^2)\left(1 + \frac{2|A_1| \cdot |A_2|}{|A_1|^2 + |A_2|^2} cos(\Delta\phi)\right) \tag{3}$$

where $\Delta\varphi = 2\pi\Delta n \cdot L/\lambda$ is the phase difference between both MZI arms. Replacing beam amplitudes by the intensities we receive:

$$I_{out} = (I_1 + I_2)\left(1 + \frac{2\sqrt{I_1 I_2}}{I_1 + I_2} cos(\Delta\phi)\right) \tag{4}$$

where factor

$$V = \frac{2\sqrt{I_1 I_2}}{I_1 + I_2} \tag{5}$$

also known as a visibility defines the beam intensity ratio, $I_1$ and $I_2$, between both MZI arms while $\Delta\varphi$ corresponds to the phase difference between them that, for equal length of the interferometer arms, is proportional to the difference in the mode effective indices between two propagating modes, $\Delta n$.

For an equal beam intensity ratio between both MZI arms, i.e., $I_1 = I_2 = 1/2 I_{inp}$ where $I_{inp}$ is the input intensity at the entrance of the MZI, the visibility $V$=1 and the absolute value of a transmission from the MZI, that can be simplified using the trigonometric function of the double-angle formula $cos(2\alpha) = 2 \cdot cos^2(\alpha)-1$, is proportional to the phase difference between both MZI arms according to the equation:

$$\frac{I_{out}}{I_{inp}} = cos^2\left(\frac{\Delta\phi}{2}\right) \tag{6}$$

In consequence, the MZI-based sensor exhibits at the output the transmission dips and peaks that correspond to destructive and constructive interference of light propagating along the sensing and reference arms.

For an optical sensor operating based on the resonant structures, the sensitivity is defined by the amount of wavelength shift caused by the effective index change of the propagating mode along the sensing waveguide due to a change in the ambient refractive index. For a proposed MZI-based sensor the wavelength shift is caused by the change of the effective index of the propagating plasmonic mode due to its exposure to the medium located in a close proximity of the metallic electrode supporting the propagating mode.

The sensitivity of the sensors is determined by the ratio of the resonance shift of wavelength to the refractive index change and can be calculated using the following formula:

$$S = \frac{d\lambda}{dn_{liq}} = \frac{d\lambda}{dn_{eff}} \frac{dn_{eff}}{dn_{liq}} \tag{7}$$

where $\lambda$ is the wavelength of the optical signal, $n_{liq}$ is the refractive index of the considered liquid and $n_{eff}$ is the mode effective index in the plasmonic waveguide. Here, the first term $d\lambda/dn_{eff}$ describes the resonant wavelength shift produced by the MZI sensor as a result of the effective refractive index change of sensing waveguide which is depended on the architecture of the sensor, while the second term $dn_{eff}/dn_{liq}$ describes the sensitivity of the sensing waveguide, which is proportional to the optical confinement factor $\Gamma$ of covering material (liquid) under sensing. The optical confinement factor $\Gamma$ is defined as the power confined in particular area divided by the total power produced by a waveguide. However, this is only true for sensing of chemical or physical quantities which are homogeneously distributed in the evanescent field of the waveguide (homogeneous sensing mechanism). In the case of sensing of ultrathin chemical, physical or biological layers of thickness much lower than a wavelength of light that are immobilized on the surface of a waveguide or in very close proximity of it, the sensitivity is defined as the rate of change of the modal effective index of the propagating mode versus the dielectric load term that is defined by a thickness of such a layer and the dielectric function difference between a thin layer and the cover material, here liquid (surface sensing mechanism). The sensitivity of the surface sensing mechanism can be maximized by increasing the electric field enhancement in a close proximity of the waveguide what can be achieved by plasmonic waveguide arrangements. Furthermore, it is enhanced in a symmetric arrangement, i.e., when the refractive index of the material under test, here liquid, is the same in both arms of the Mach-Zehnder Interferometer. It means that the refractive index of the liquid that is in contact with one of the MZI arms is the same as the refractive index of a liquid that is in contact with the second arm of the MZI. In the sensor proposed here it is achieved automatically as the same material/liquid under test fills uniformly the entire space between metal electrodes in static conditions, i.e., for unbiased case, thus, both of the metal electrodes (e.g. **601, 602** in **Fig. 6**) are in contact with the same material/liquid (e.g. **605, 606** in **Fig. 6**).

The proposed design can utilize both sensing mechanisms depending on the biasing conditions i.e., homogeneous sensing mechanism for either the unbiased case (no voltage applied between metal electrodes) (e.g. **607** in **Fig. 6**) or in the absence of the ion-exchange layer (e.g. **708** in **Fig. 7**) and surface sensing mechanism for either a biased condition (voltage applied between metal electrodes) (e.g. **608** in **Fig. 6**) or in the presence of the ion-exchange layer/membrane (e.g. **707** in **Fig. 7**). Thus, it brings a lot of flexibility in terms of a design of photonic integrated sensors.

Referring to Figs. 6 and 7, in the case of the **surface sensing mechanism,** the ions (or biological molecules) **606** can be placed in the maximum electric field of the propagating mode **611** i.e. in the metal-sensing medium interface, thanks to the applied DC voltage **608** between the metal electrodes **601, 602** that forces ions **606** to move in a direction of one of the metal electrode edges (electrode edge **604** in this ) or as a result of the ion-exchange layer **707** on one of the sides of the metal electrodes (electrode side **704** in this case). Those metal electrodes **601, 602** serve both as the electrical contacts and plasmonic slot waveguides that support propagating modes **610, 611.** In comparison, in other proposed waveguide arrangements the ions are placed randomly in the entire volume of a liquid, thus, the spatial overlap between the evanescent field of the plasmonic or photonic mode and the analyte under test is much lower as only small amount of ions is placed in the maximum field of the propagating mode while a definite majority is placed far away from a metal surface where the electric field of the propagating mode is much lower.

The separation of ions in a medium, here liquid, depends on the DC electric field value **609** or the presence of the ion-exchange layer/membrane **707,** thus, the phase shit between both MZI arms of the plasmonic slot waveguide can be controlled by the applied DC voltage **608** or the presence of the ion-exchange layer **707.** This brings another possibility to control the transmission from the sensor, and thus, sensitivity of the sensor.

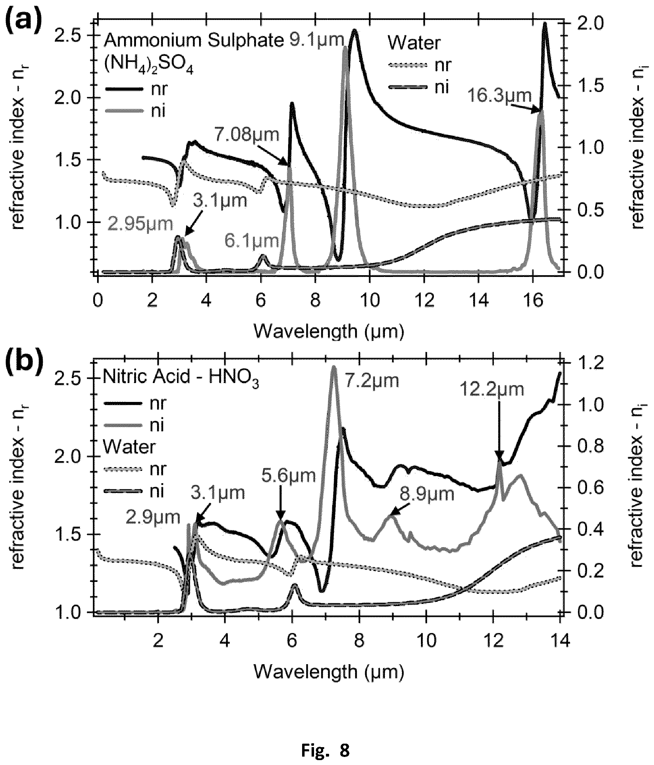

The operating principles of the proposed sensor arrangement were initially simulated at a telecom wavelength of 1550 nm, however, the real device should operate at the wavelengths that correspond to the maximum absorption peaks for a given chemical compounds as shown in **Fig. 8.** Since most of the chemical compounds show the characteristic absorption peaks in the mid-infrared (MIR) wavelength, the proposed sensor should be designed for these specific wavelengths.

As illustrated in **Fig. 8,** the chemical and biological compounds show characteristic absorption spectra defined by their absorption peaks that can serve as a so-called finger print for a given compound. The maximum absorption originates from stretching vibrations and bending modes of the molecules that constitute a given compound. From the point of view of the photonics, the maximum absorption corresponds to the highest optical losses and, simultaneously, the highest gradient of the refractive index change, thus, it can be used for sensing.

### Influence of the absorption spectrum of the analyte on the choice of wavelength spectrum

Each analyte shows characteristic absorption spectrum with the maximum absorption that originates from stretching vibrations and bending modes of the molecules that constitute a given analyte. The maximum absorption corresponds to the highest losses, thus, the material is characterized by the highest imaginary part of the permittivity. In consequence, a simple straight waveguide that operates on the basis of the change in transmission of light through the analyte as a consequence of the high absorption of an analyte can be considered. However, at the same time, the maximum absorption corresponds to the highest change of the real part of permittivity what enables a construction of sensors that operates based on the refractive index change what is directly corelated with the phase change of the propagating mode through the analyte. For this second scenario, the resonant devices such as Mach-Zehnder Interferometers (MZIs) or ring resonators (RRs) can be considered. To date, numerous studies have showed that MZI is more tolerant of minor inaccuracies during the production process while it can offer higher sensitivity values compared to RR-based sensors. Up to now, several different MZI arrangement were proposed to maximize sensitivity, minimize the footprint and the overall complexity. While most of the proposed MZI sensors operate based on the two spatially separated arms with one serving as a sensing arm and the second as a reference arm, the more sophisticated designs were proposed as well. One of them, known as bi-modal interferometers, utilizes a single-path interferometer that exploit modes with different properties propagating in the same physical channel, i.e., on top and bottom layer of the metal stripe, that interfere together at the termination of the metal stripe. In this arrangement the bottom metallic surface plays the role of the reference arm of the interferometric structure while the top metallic surface is exposed to the liquid under test and, thus, serves as the sensing arm. However, such sensor design requires very precise alignment of the metal stripe in relation to the input and output waveguide and even a small deviation from an optimal alignment can cause a huge change in a coupling ratio between both modes. Furthermore, for each wavelength under interest, the bi-modal structure should be redesigned as it will require a different thickness or even material of the bottom layer to match the refractive index of an analyte to a desired wavelength. In consequence, it will require realignment of the metal stripe in terms of the input and output waveguide. As a result, this sensor design is impractical for application where a broad spectrum of wavelengths is considered.

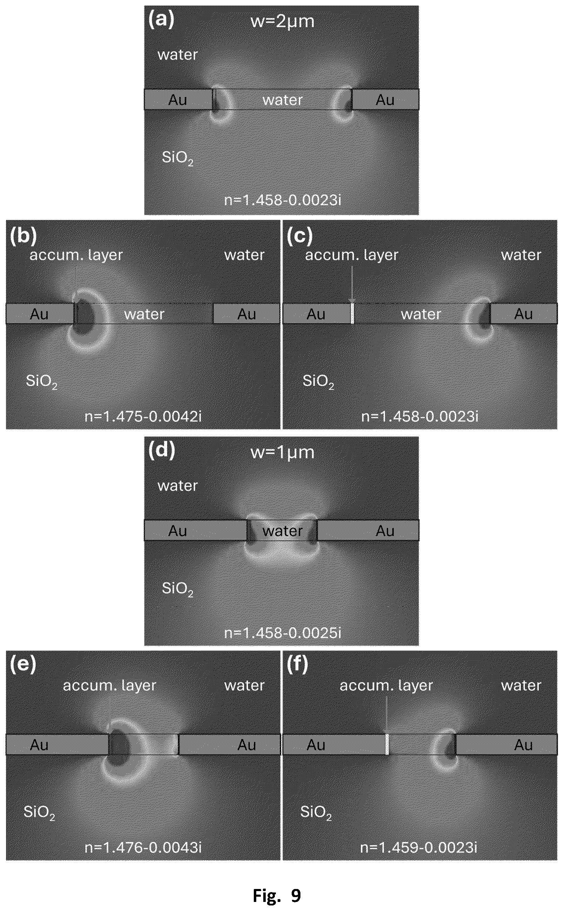

Reference is made now to **Fig. 9** showing cross-sections of a simulated in-plane optical electric field component of the propagating TE mode of a plasmonic slot waveguide with a thin layer of ions/molecules accumulated in one metal-water interface.

The simulations were performed for gold (Au) electrodes with a separation between them of 2 $\mu$m (**Fig. 9a, b, c**) and 1 $\mu$m (**Fig. 9d, e, f**). The thickness of the Au electrodes was kept constant at 300 nm to match the thickness of the SiN photonic waveguide, which was assumed to couple light to a considered sensor. In addition, the simulations were performed for water as a test material with some ions in it with a refractive index corresponding to $n$=1.8. The water with a mixture of ions was placed directly in the gap and on top of the MIM plasmonic structure.

At zero applied voltage, the SPP modes at both edges of the metal electrodes are the same, so they couple together to provide one MIM plasmonic mode with a complex mode effective index $n_{eff}$=1.458+0.0025·$i$ for a metal separation of 2 $\mu$m and $n_{eff}$=1.458+0.0028·$i$ for a metal separation of 1 $\mu$m. Those values correspond to the attenuation losses of 0.09 dB/$\mu$m and 0.10 dB/$\mu$m for metal separations of 2 $\mu$m and 1 $\mu$m, respectively. The higher losses for a smaller separation between metal electrodes are related to the higher absorption by metal electrodes, since more electric field penetrates metal resulting in higher attenuation. In such a case, the beam intensity ratio in both MZI arms is the same and both of the SPP modes encounter the same phase shift and attenuation, so there is no phase shift between both MZI arms. Consequently, the symmetric structure without applied DC volage behaves as a nonresonant structure (**Fig. 9a** and **d**). As it has been previously shown experimentally, the transmission spectrum of such a MIM waveguide shows a flat response over a broad spectral range and depends only on the length of the plasmonic MIM waveguide. However, when the DC voltage is applied between metal electrodes the ions start to move towards one of the metal electrodes (**Fig. 9b, c, e, f**), i.e., the negative ions drift towards the metal electrode with a positive DC voltage while the positive ions, if present in the liquid at the same time, drift towards the metal electrode with a negative DC voltage applied to it. As a result, ions settle on one of the metal electrodes while the other one remains free of ions (**Fig. 9b, c, e, f**). This introduces an asymmetry between the two arms of the MZI, which depends on the ion concentration on the electrode and the optical properties of the ionic compounds for a given wavelength of interest. Assuming the accumulation layer thickness of 50 nm that is created when ions move towards one of the metal electrodes and the refractive index of ions under an interest of $n$=1.8, the mode effective index for the SPP mode associated with this interface was calculated to be $n_{eff}$=1.475+0.0042·$i$ while for a second interface without ions it was calculated to be $n_{eff}$=1.458+0.0023·$i$ which translates into propagation losses of 0.15 dB/$\mu$m and 0.08 dB/$\mu$m, respectively (**Fig. 9**). Based on a provided data, the $L$=45 $\mu$m long MIM waveguide is required to provide a $\pi$ phase shift between both arms of the interferometer with a $V$ factor (visibility) calculated for this length of MIM waveguide at $V$≈0.94, which translates to the extinction ratio ($ER$=$I_{max}$/$I_{min}$) exceeding an impressive value of 15 dB. Furthermore, the calculation has shown that for a 100 $\mu$m long MZI waveguide arranged under the same conditions, a phase shift of more than $2\pi$ between both MZI arms is possible with the visibility exceeding $V$=0.75 which gives an extinction ratio $ER$=8.5 dB.

As the concentration of ions under test in a liquid increases, more ions in the liquid move to one of the metal electrodes under applied DC voltage. In consequence, the concentration of ions on this metal electrode increases what enhances an interaction of ions with the electromagnetic field supported by one of the propagating SPP modes.

Simulations performed for a wavelength of light $\lambda$=1550 nm have shown that only 45 $\mu$m long plasmonic slot waveguide is required to ensure a full $\pi$ phase shift between both plasmonic slot arms made of gold with a thickness of 400 nm for a water sample with the refractive index n=1.332 placed inside a slot and above it with an analyte inside water that accumulate at one of the metal-water interference under applied voltage (**Fig. 9**). The thickness of an accumulation layer was assumed at only 50 nm while an analyte refractive index under test at n=1.8. It was additionally assumed that entire structure was placed on a low refractive index $SiO_2$ material with the refractive index n=1.45. It was observed that reduction of a slot width from w=2 $\mu$m to w=1 $\mu$m does not significantly influence the propagation constants of the modes on both metal-water interferences. Simultaneously, it was observed that reduction of the analyte refractive index (accumulation layer) to n=1.7 does not degrade the performance of the sensor as the V parameter, that defines a drop in the transmission level under propagation through the MZI, decreases only slightly from V=0.95 to V=0.83 as the slot length increases to

L=82 $\mu$m that is required to provide a full $\pi$ phase shift between both arms of the plasmonic slot waveguide. And most importantly, the above simulations have shown that it is highly desired to work in specific wavelengths for which an analyte exhibits a maximum absorption and, simultaneously, the highest change in its real part of the refractive index (permittivity), as such materials are very sensitive even to small changes of its properties.

To provide a $\pi$ phase shift between both arms of the slot waveguide before the ions separation by the external voltage, one of the arms can be designed with a certain curvature while the second arms can be keep straight. In this scenario, the longer arms provide more phase accumulation just to provide a $\pi$ phase shift between both arms.

The proposed sensor is capable of detecting a broad range of common ions present in water and aqueous solutions including nitrites ( $NO_2^-$ ), nitrates ( $NO_3^-$ ), phosphates ( $PO_4^{3-}$ ), and ammonium ions ( $NH_4^+$ ), among others. This makes it an ideal candidate for monitoring water pollutants. To fully leverage the proposed design, it is essential to operate within the spectral range where ions exhibit the highest absorption peaks, thereby maximizing sensitivity. The mid-IR band is of great significance, encompassing the primary absorption bands of the majority of chemical and biological molecules, as well as the fingerprint region (7-20 $\mu$m). This makes it a crucial component in spectroscopic sensing. The mid-IR absorption of most chemical compounds is at least 100 times stronger than their absorption bands in the near-IR despite the lower energy they carry in comparison to their visible or near-IR counterparts. This makes them more susceptible to thermal fluctuations.

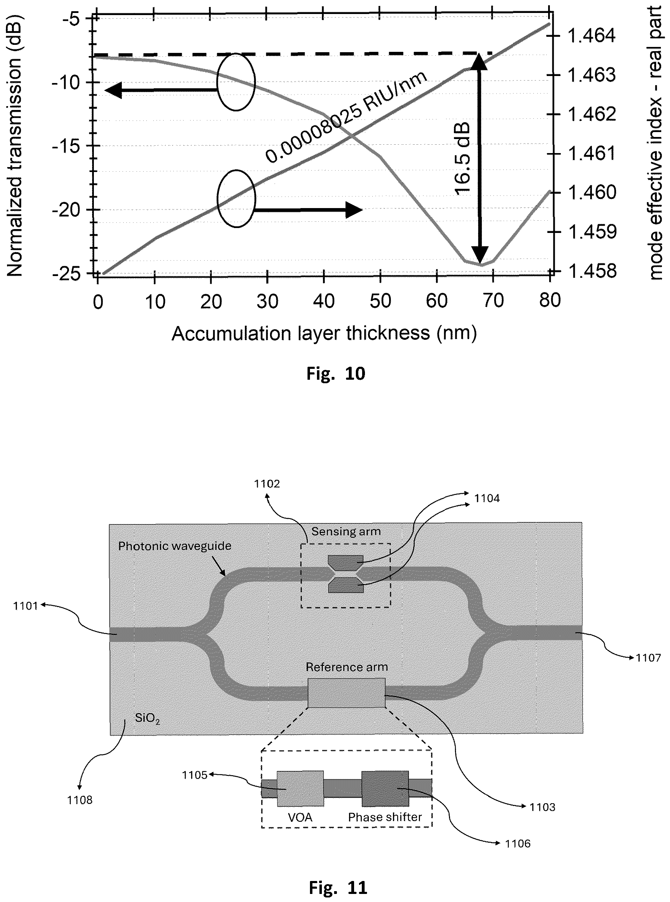

The proposed sensor design is very sensitive to the presence of ions in a liquid solution thus it can operate even in spectral range that is far away from the desired wavelength range due to the surface sensing mechanism implemented in the proposed structure. The 2D eigenmode simulation performed at wavelength of 1550 nm revealed that for an Au metal electrode separation of 2 $\mu$m deposited on SiO$_2$ ($n_{SiO2}$=1.45) and for water filling a gap between metal electrodes, that is described by a complex refractive index $n_{water}$=1.318+9.8625$\cdot$10$^{-5}$$i$ and NH$_4^+$ ions ($n_{NH4}$=1.517) bounded to one of the metal electrodes that thickness changes with the concentration of ions in water, the surface SPP sensitivity of this mode was found equal to 0.00008025 RIU/nm (as illustrated in **Fig. 10)** according to the equation

$$S_s = \frac{\delta n_{eff}}{\delta t} \tag{8}$$

Here, $n_{eff}$ represents the effective index of the SPP mode with bounded ions and $t$ is the thickness of the ions layer. In consequence, a sensor surface sensitivity was calculated at 12460 nm/RIU what is at least **3 times** higher than sensitivities previously reported for a bi-modal sensor configuration and more than **one order** of magnitude (10 times) higher than for other proposed sensors known in the art.

Simultaneously, the extinction ratio (ER) between two different conditions, no ions in water and 68 nm thick layer of ions bounded to one of the metal electrodes, was calculated at 16.5 dB (**Fig. 10**). For this thickness the difference in phase between both MZI arms reaches $\pi$ and light transmitted through a sensor is minimum. Further increases in the ions thickness will lead to further increases in phase difference between both MZI arms what results in a further increase in the light transmitted from the interferometer.

**Sensor integration**

The sensor is very often understood as a separate component that detects any changes in the surrounding environment and so on. However, from a formal point of view, the sensor is understood as a complete system that allows to perform a complete set of measurements without the need for any additional components. Thus, a sensor must comprise a source, a transducer, and a detector. Consequently, depending on the requirements, all components can be integrated on-chip or taken off-chip with only coupling to the transducer chip as it is application specific. In a second scenario, it will require efficient coupling arrangements that can be divided into grating and end-fire coupling (butt-coupling). Both of them can be implemented with optical fibers with a proper alignment to maximize coupling efficiency, or free space techniques. The choice of a specific arrangement is mostly defined by applications and costs. For example, medical applications require the off-chip sensors due to the risk of cross-contamination as a transducer would be disposable after a single use. It is impractical from this perspective to integrate a costly laser source onto the same chip. On the other hand, the environmental sensors such as gas, liquid and heat sensors, can be reused thus they can be integrated on a single chip. On-chip integration of sensors brings some valuable benefits as it provides stability to the system that makes it tolerant to any vibration that can appear during measurements. For example, atmospheric gas sensors would greatly benefit from improved vibration tolerance and reduced footprint associated with integration.

According to the details provided above, the proposed sensor can contain all components i.e., a light source, a transducer and a photodetector integrated on one chip or can contain all components integrated off-chip with one or more components on one chip and other component(s) or another chip or each component on a separated chip or in any other configuration. Furthermore, the proposed sensor can contain other optical or electrical components such as, for example, modulators, attenuators, phase shifters, and so on. As a light source one should consider broadband sources, LEDs, VCSELs or other optical sources. The light sources and photodetectors are arranged at the input and output of the sensor.

According to a further embodiment of the system according to the present invention which is presented in **Fig. 11,** it can comprise a photonic optical Mach-Zehnder interferometric sensor with a plasmonic transducer **1104** arranged in the so-called sensing arm **1102** of the MZI and a variable optical attenuator (VOA) **1105** and phase shifters **1106** in the second branch, so-called reference arm **1103** of the MZI, in order to optimally balance the sensor and thus, its sensitivity. The light couples in and out to such a sensor from a photonic waveguide **1101, 1107** deposited on a low refractive index substrate **1108** to minimize overall propagation losses.

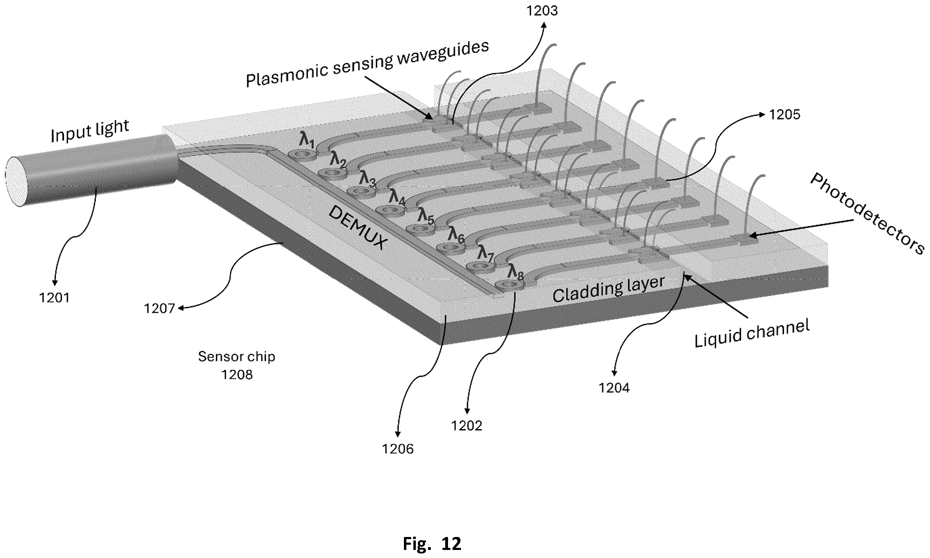

Reference in made now to a further embodiment illustrated in **Fig. 12** illustrating an apparatus comprising a combination of multiple plasmonic sensing waveguides. As most of the analytes are defined by multiple absorption peaks (**Fig. 8**), the proposed sensor can contain arrays of integrated optical sources or a broadband light source, an optical fiber **1201** that couples light to a sensor chip **1208,** a wavelength division multiplexer (DEMUX) **1202** that separates the specific wavelengths, and optical detectors **1205** that are arranged at the input and output of the apparatuses.

According to this further embodiment of the apparatus according to the present invention, the apparatus comprises a fluidic channel **1204** which is created on a plasmonic transducer **1203** by a deposition of a cladding layer **1206** made of a low refractive index material on top of the sensor chip except of the transducer elements **1203** or arrays of plasmonic transducers **1203** to flow a predetermined solution/analyte on the plasmonic transducer element **1203** or arrays of transducer elements. The chip is manufactured on substrate **1207** made of a low refractive index material. The mentioned materials of a low refractive index can be, e.g., $SiO_2$, polymers and other materials know in the art. One skilled in the art will understand that the materials used should be selected so as to ensure a sufficiently high refractive index contrast between particular layers and/or components (typically between waveguides and surrounding structures).

### Types of sensors

Depending on the applications, the sensors can be divided in the main four groups:

- Gas sensors
- Liquid sensors
- Heat sensors
- Biomedical sensors

The gas sensors can analyze industrial emissions for greenhouse and toxic gases while liquid sensors can analyze the water pollutants and deep-sea dissolved greenhouse gases. Compared to the gas sensors, the liquid sensors need to address broad absorption features in much denser medium what requires broader sensors, however, their footprint can be smaller as they require orders of magnitude lower absorption length. On the contrary, the heat sensors can be used for military purposes to detect and identify heat signatures or for civilian purposes to monitor a heat emission for building control.

The biomedical sensors can be divided into two main categories - one for detection of a particular analyte such as a protein or a drug that utilizes a single wavelength measurements (narrowband spectrum) and second for investigation of system level changes that utilizes the entire mid-IR spectrum (wideband spectrum). The application of biomedical sensors spreads from a drug monitoring through a protein sensing for a cancer diagnosis, sepsis, SARS-CoV-2 up to malaria, just to mention only few of them.

According to the embodiment of the device according to the present invention, a detection channel formed with only one transducer or arrays of transducers **1203** can contain any fluids, gases, specific biological and/or chemical substances and/or molecules. The detection channel **1204** can contain any antibodies immobilized either on the metallic surface of the sensor or a substrate inside a channel or below a channel to catch specific antigens that are under the interest. Furthermore, one or both of the electrodes of the transducers **1203** can be coated with a so-called preconcentration material **606** (also known as ion-selective material) to enhance sensitivity and selectivity by selectively absorbing and concentrating the analytes within the sensing volume (**Fig. 7**). The preconcentration material can be selected from the group comprising:

- ion exchange resin;
- metal-oxides; and
- metal-organic frameworks (MOFs).

### Coupling scheme to the plasmonic sensor

Up to now, three efficient mechanisms of coupling light from a photonic waveguide to an active part of the sensor can be considered and all of them can be implemented in the proposed device - in-plane waveguide coupling, evanescent coupling from a photonic waveguide buried in a substrate below or grating coupling.

- Butt-coupling arrangement

The light can effectively couple from photonic waveguide to the plasmonic slot waveguide through inverse taper in the photonic waveguide tip as shown in **Fig. 1**. As it has been previously shown for such arrangement, the coupling efficiency only slightly depends on the plasmonic slot height, however, the thicker slot provides slightly higher coupling efficiency. More importantly, the coupling efficiency depends strongly on the gap width and increases as the gap width decreases. In consequence, the coupling efficiency below 0.8 dB can be achieved for a small plasmonic gap.

- Evanescent coupling arrangement

In this coupling arrangement (**Fig. 2**), the optical signal is guided by passive photonic waveguides that are buried in a substrate below, and evanescently coupled to the active section of the sensor i.e., plasmonic slot waveguide. As it has been previously in the art, coupling efficiency of up to 90 % can be expected from Si photonic waveguide to plasmonic slot waveguide for a perfectly fabricated device that can be made with the state-of-the-art Si photonic fab. It can be achieved if the waveguide stub below the plasmonic section is thinned down below 60 nm or completely removed, optimal coupling can be achieved.

- Grating coupling arrangement

The light couples to a sensor through either metallic or dielectric grating which adiabatically funnels the mode from the tapered region to the plasmonic slot sensor section (**Fig. 3**). The coupling efficiency to the gap surface plasmon as high as 50 % can be achieved with a proper design of gratings.

According to the embodiments of the device according to the present invention presented and explained herein, the proposed sensor can be designed in any of the above arrangements and can provide an efficient light delivery to the sensors under consideration.

It will be understood that the present invention is not limited to the presented embodiments or examples but the scope of the invention is defined by the attached claims.

## Claims

1.   A photonic sensor device comprising at least one optical interferometric sensor, wherein a plasmonic slot waveguide is incorporated as a transducer element planar integrated on photonic waveguides, wherein the device comprises:

   - a set of high refractive index photonic waveguides (**101, 109**) arranged on a low refractive index substrate, sandwiched between a low refractive index substrate (**111**) and a low refractive index cladding, or buried in a low refractive index substrate (**207**);
   - a plasmonic slot waveguide that confines light propagation through coupling to Surface Plasmon Polaritons (SPP) at metal-analyte interfaces; wherein the plasmonic slot waveguide is defined by opposite inner edges of electrodes (**104, 105**), and wherein said plasmonic slot waveguide forms a Mach-Zehnder interferometer, acting as a plasmonic slot Mach-Zehnder interferometric sensor, with two plasmonic modes propagating on the opposite inner edges of the electrodes (**104, 105**) that constitute the plasmonic slot waveguide and that define the Mach-Zehnder Interferometer arms;

   wherein the space between and/or on the electrodes (**104, 105**) is intended for an analyte to be tested;

   - optical coupling structures (**102, 107**) at both ends of the plasmonic slot Mach-Zehnder interferometric sensor acting as optical input/output from and to photonic waveguides (**101, 109**):
   - an optical splitter (**103**) and an optical combiner (**108**) for optical splitting at a first junction (**103**) of said plasmonic slot Mach-Zehnder interferometric sensor and optical combining at a second junction (**108**) of said plasmonic slot Mach-Zehnder interferometric sensor; and
   - a DC voltage source (**106; 508**) that applies a bias voltage to the metal electrodes (**104**, **105**).

2.   The device according to claim 1, wherein the material of the photonic waveguides (**101, 109**) is selected from Si, SiN, Ge, TiO$_2$ or Al$_2$O$_3$ while the material of the substrate (**111**) and/or cladding is selected from SiO$_2$ or low refractive index polymers, depending on the light wavelength used and specific requirements.

3. The device according to claim 1, wherein the arms (**505, 509**) of the Mach-Zehnder interferometer defined by said plasmonic slot waveguide have different lengths, particularly by a curvature of at least one of the arms, with one arm (**509**) being straight while the other arm (**505**) being curved with a different curvature degree, or with both arms (**504, 505**) being curved with the same degree, or with both arms being curved but with different degree.

4. The device according to claim 1, wherein said bias voltage applied by the DC voltage source (**106**) is applied between both electrodes (**104, 105**) or between one of the electrodes (**104, 105**) and the substrate (**111**) on which the electrode is fabricated.

5. The device according to claim 1, wherein said plasmonic slot waveguide (**104, 105**) is made of gold (Au), silver (Ag), aluminium (Al), copper (Cu), materials from the transition metal nitrides group including titanium nitride (TiN), zirconium nitride (ZrN), materials from the transparent conductive oxides group including Aluminium zinc oxide (AZO), indium tin oxide (ITO), gallium zinc oxide (GZO), cadmium oxide (CdO) or other CMOS compatible plasmonic materials and metals.

6. The device according to claim 1, wherein each of the electrodes (**104, 105**) that constitute the plasmonic slot waveguide is made of different plasmonic materials including gold (Au), silver (Ag), aluminum (Al), copper (Cu), materials from the transition metal nitrides group including titanium nitride (TiN), and zirconium nitride (ZrN), materials from the transparent conductive oxides group including Aluminium zinc oxide (AZO), indium tin oxide (ITO), gallium zinc oxide (GZO), cadmium oxide (CdO) or other CMOS compatible plasmonic materials and metals.

7. The device according to claim 1, wherein light coupling between the photonic waveguides (**101, 109**) and the plasmonic slot waveguide (**104, 105**) is realized through an in-plane waveguide coupling arrangement, where the photonic waveguide (**101, 109**) is tapered (**102**) or straight, an evanescent coupling arrangement with the photonic waveguide (**201, 206**) buried in the substrate (**207**) below, or a grating coupling arrangement directly by a fiber (**301, 306**) through a grating coupler (**302, 305**).

8. The device according to claim 1, wherein one or both electrodes (**701, 702**) have their inner sides (**703, 704**) coated with a preconcentration material (**707**) which is selected from the group including:

   an ion exchange resin;
   metal-oxides; and
   metal-organic frameworks (MOFs).

9. The device according to claim 1, wherein any antibodies are immobilized either on a metallic edge of one electrode (**504, 505**) or on metallic edges of both electrodes (**504, 505**) or directly on a substrate between the metal electrodes (**504, 505**) to catch specific antigens for a biological detection.

10. The device according to claim 1, wherein the space for analyte (**605**) to be tested is adapted for delivery any form of the analyte, including gas, liquid, fluid or any other biological sample.

11. The device according to claim 1, where one or both electrodes (**601, 602**) collect the separated chemical, biochemical, biological substances and/or molecules or other physical quantities (**606**) present in liquids, fluids or gases or any biological samples (**605**).

12. The device according to claim 1, wherein plasmonic slot waveguide (1104) is incorporated into a sensing arm (**1102**) of a photonic Mach-Zehnder Interferometer, the reference arm (**1103**) of which has incorporated a variable optical attenuator (VOA) and a phase shifter (**1106**) to balance the reference arm (**1103**) in terms of the sensing arm (**1102**);

13. An apparatus comprising an array of devices as defined in one of claims 1-11 for concurrently detecting multiple substances on the same chip (**1208**), wherein said apparatus comprises multiple plasmonic slot waveguides (**1203**) arranged in individual branches wherein each plasmonic slot waveguide (**1203**) uses a separate wavelength out of a number of wavelengths that are simultaneously injected through a fiber (**1201**) in the sensor chip (**1208**), wherein each plasmonic slot waveguide (**1203**) also comprises an optical filter (**1202**) at the input of its branch and after input splitter to select its wavelength of operation from the incoming optical signals (**1201**), wherein said optical filters (**1202**) are particularly ring resonators (**1202**).

14. The apparatus according to claim 13, wherein integrated optical sources, light-emitting diodes (LED), vertical-cavity surface-emitting lasers (VCSEL), broadband sources or other optical sources and optical photodetectors (**1205**), are arranged at the input and output of the sensor chip (**1208**), respectively, wherein the optical source (**1201**) and the optical photodetectors (1205**)** are integrated using flip-chip or wafer bonding or die bonding or epitaxial growth methods above a grating coupler or on the same level of the photonic waveguide.

15. The apparatus according to claim 13, wherein integrated optical sources, LEDs, VCSELs, broadband sources or other optical sources (**1201**) and/or optical photodetectors (**1205**), are integrated on a separated chip or chips.

16. The apparatus according to claim 13, wherein arrays of integrated optical sources (**1201**) and optical detectors (1205**)** are arranged at the inputs and outputs of a single sensor chip (**1208**), wherein the optical sources (**1201**) and photodetectors (**1205**) are integrated using flip-chip or wafer bonding or die binding or epitaxial growth methods

17. The apparatus according to claim 13, wherein it comprises a fluidic channel, a liquid channel or a gas channel (**1204**), which is formed on the surface of the plasmonic slot waveguides (**1203**) to flow a predetermined solution/analyte on the plasmonic slot waveguides (**1203**).

**Fig. 1**

**(a)**

202    201    203

207

waveguide

SiO$_2$

**(b)**

204

MIM waveguide

202

electrode 1

photonic waveguide

light in

201

electrode 2

photonic waveguide

light out

SiO$_2$

205

203

206

207

**Fig. 2**

306

301

303

MIM waveguide

fiber

electrode 1

fiber

electrode 2

SiO$_2$

304

305

302

307

**Fig. 3**

Fig. 4

**Fig. 5**

Fig. 6

Fig. 7

**(a)** Ammonium Sulphate 9.1μm $(NH_4)_2SO_4$ — nr, ni; Water — nr, ni; 7.08μm, 16.3μm, 2.95μm, 3.1μm, 6.1μm

**(b)** Nitric Acid - $HNO_3$ — nr, ni; Water — nr, ni; 7.2μm, 12.2μm, 5.6μm, 2.9μm, 3.1μm, 8.9μm

**Fig. 8**

**Fig. 9**

**Fig. 10**

**Fig. 11**

Fig. 12

Europäisches
Patentamt

European
Patent Office

Office européen
des brevets

# EUROPEAN SEARCH REPORT

**Application Number**

EP 25 15 5292

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | FOTIADIS K ET AL: "Single-arm Interferometric Plasmonic Sensor integrated on a cladded polymeric photonic platform", 2024 CONFERENCE ON LASERS AND ELECTRO-OPTICS (CLEO), OPTICA, 5 May 2024 (2024-05-05), pages 1-2, XP034738892, DOI: 10.1364/CLEO_SI.2024.SF1A.7 [retrieved on 2024-10-29] * abstract * * figure 1 * | 1-17 | INV. G01N21/45 G01N21/552 G01N21/77 G01N33/543 G02B6/122 ADD. G01B9/00 |
| A | XU SUN ET AL: "High-sensitivity liquid refractive-index sensor based on a Mach-Zehnder interferometer with a double-slot hybrid plasmonic waveguide", OPTICS EXPRESS, vol. 23, no. 20, 22 September 2015 (2015-09-22), page 25688, XP055413468, DOI: 10.1364/OE.23.025688 * abstract * * figures 1, 4, 5 * ----- -/-- | 1-17 | |
| | | | TECHNICAL FIELDS SEARCHED (IPC) G01N |

The present search report has been drawn up for all claims

| Place of search | Date of completion of the search | Examiner |
|---|---|---|
| Munich | 23 June 2025 | Sauerer, Christof |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 3

Europäisches Patentamt
European Patent Office
Office européen des brevets

# EUROPEAN SEARCH REPORT

**Application Number**

EP 25 15 5292

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ZHU SHIYANG ET AL: "Theoretical investigation of silicon MOS-type plasmonic slot waveguide based MZI modulators", OPTICS EXPRESS, vol. 18, no. 26, 17 December 2010 (2010-12-17), page 27802, XP093287069, US ISSN: 1094-4087, DOI: 10.1364/OE.18.027802 Retrieved from the Internet: URL:https://opg.optica.org/directpdfaccess /a654d560-1f7d-43f8-8a2569127af99dbe_20900 9/oe-18-26-27802.pdf?da=1&id=209009&seq=0& mobile=no> * abstract * * figure 1 * | 1-17 | |
| A,D | US 2020/003696 A1 (PLEROS NIKOLAOS [GR] ET AL) 2 January 2020 (2020-01-02) * figures 1, 10 * | 1-17 | |
| A | MANOLIS ATHANASIOS ET AL: "Bringing Plasmonics Into CMOS Photonic Foundries: Aluminum Plasmonics on Si3N4 for Biosensing Applications", JOURNAL OF LIGHTWAVE TECHNOLOGY, IEEE, USA, vol. 37, no. 21, 1 November 2019 (2019-11-01), pages 5516-5524, XP011756819, ISSN: 0733-8724, DOI: 10.1109/JLT.2019.2937454 [retrieved on 2019-10-31] * abstract * * figures 1, 2, 11 * | 1-17 | TECHNICAL FIELDS SEARCHED (IPC) |

-/--

The present search report has been drawn up for all claims

| Place of search | Date of completion of the search | Examiner |
|---|---|---|
| Munich | 23 June 2025 | Sauerer, Christof |

EPO FORM 1503 03.82 (P04C01)

Europäisches
Patentamt

European
Patent Office

Office européen
des brevets

# EUROPEAN SEARCH REPORT

**Application Number**

EP 25 15 5292

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | NIELSEN MICHAEL P. ET AL: "Adiabatic Nanofocusing in Hybrid Gap Plasmon Waveguides on the Silicon-on-Insulator Platform", NANO LETTERS,20200311AMERICAN CHEMICAL SOCIETY, US, vol. 16, no. 2, 15 January 2016 (2016-01-15), pages 1410-1414, XP093287137, ISSN: 1530-6984, DOI: 10.1021/acs.nanolett.5b04931 Retrieved from the Internet: URL:https://pubs.acs.org/doi/pdf/10.1021/acs.nanolett.5b04931> * abstract * * figures 1, 3 * | 1-17 | |
| A,D | FOTIADIS KONSTANTINOS ET AL: "High-Sensitivity Bimodal Plasmo-Photonic Refractive Index Sensor", ACS PHOTONICS, vol. 10, no. 8, 7 August 2023 (2023-08-07), pages 2580-2588, XP093287424, ISSN: 2330-4022, DOI: 10.1021/acsphotonics.3c00290 Retrieved from the Internet: URL:https://pubs.acs.org/doi/pdf/10.1021/acsphotonics.3c00290> * abstract * * figure 1 * | 1-17 | TECHNICAL FIELDS SEARCHED (IPC) |

The present search report has been drawn up for all claims

| Place of search | Date of completion of the search | Examiner |
|---|---|---|
| Munich | 23 June 2025 | Sauerer, Christof |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

page 3 of 3

## ANNEX TO THE EUROPEAN SEARCH REPORT
## ON EUROPEAN PATENT APPLICATION NO.

EP 25 15 5292

This annex lists the patent family members relating to the patent documents cited in the above-mentioned European search report.
The members are as contained in the European Patent Office EDP file on
The European Patent Office is in no way liable for these particulars which are merely given for the purpose of information.

23-06-2025

| Patent document cited in search report | Publication date | Patent family member(s) | | Publication date |
|---|---|---|---|---|
| US 2020003696 A1 | 02-01-2020 | AU | 2018221428 A1 | 10-10-2019 |
| | | CA | 3053715 A1 | 23-08-2018 |
| | | CN | 110325840 A | 11-10-2019 |
| | | EA | 201991909 A1 | 05-02-2021 |
| | | EP | 3583406 A1 | 25-12-2019 |
| | | GR | 20170100088 A | 31-10-2018 |
| | | JP | 7212901 B2 | 26-01-2023 |
| | | JP | 2020508471 A | 19-03-2020 |
| | | KR | 20190128172 A | 15-11-2019 |
| | | US | 2020003696 A1 | 02-01-2020 |
| | | WO | 2018150205 A1 | 23-08-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2020003696 A1
- US 20110188047 A1
- US 2018340884 A1

**Non-patent literature cited in the description**

- **M. P. NIELSEN** ; **L. LAFONE** ; **A. RAKOVICH** ; **T. P. H. SIDIROPOULOS** ; **M. RAHMANI** ; **S. A. MAIER** ; **R. F. OULTON**. Adiabatic Nanofocusing in Hybrid Gap Plasmon Waveguides on the Silicon-on-Insulator Platform. *Nano Lett.*, 2016, vol. 16 (2), 1410-1414
- **O. PARRIAUX** ; **G. J. VELDHUIS**. Normalized Analysis for the Sensitivity Optimization on Integrated Optical Evanescent-Wave Sensors. *J. of Lightwave Tech.*, 1998, vol. 16 (4), 573-582
- **K. FOTIADIS et al.** High-Sensitivity Bimodal Plasmo-Photonic Refractive Index Sensor. *ACS Photonics*, 2023, vol. 10, 2580-2588
- **B. HINKOV** ; **M. DAVID** ; **G. STRASSER** ; **B. SCHWARZ** ; **B. LENDL**. On-chip liquid sensing using mid-IR plasmonics. *Front. Photonics*, 2023, vol. 4, 1213434
- **C. J. MITCHELL** ; **T. HU** ; **S. SUN** ; **C. J. STIRLING** ; **M. NEDELJKOVIC** ; **A. C. PEACOCK** ; **G. T. REED** ; **G. MASHANOVICH** ; **D. J. ROWE**. Mid-infrared silicon photonics: From benchtop to real-world applications. *APL Photon.*, 2024, vol. 9, 080901